# EUROPEAN PATENT APPLICATION

(11) **EP 3 095 877 A1**
(43) Date of publication of application: **23.11.2016**
(21) Application number: 15305758.3
(22) Date of filing: 20.05.2015
(51) Int. Cl.: C12Q 1/68

(54) **METHODS AND MEANS FOR MANAGING EXTENDED-SPECTRUM BETA-LACTAMASE (ESBL)-PRODUCING ENTEROBACTERIACEAE COLONIZATION**

(71) Applicant: Universitat de València, 46010 Valencia (ES); Fundación para el Fomento de la Investigación Sanitaria y Biomedica de la comunitat Valenciana, 46020 Valencia (ES); Centro de Investigación Biomédica en Red, 28029 Madrid (ES); Université Paris Diderot - Paris 7, 75013 Paris (FR); ASSISTANCE PUBLIQUE-HOPITAUX DE PARIS, 75004 Paris (FR)
(72) Inventor: MOYA, Andrés, 46110 Valencia (ES); LATORRE, Amparo, 46110 Valencia (ES); GOSALBES, Maria José, 46023 Valencia (ES); VAZQUEZ CASTELLANOS, Jorge F., 46022 VALENCIA (ES); ANDREMONT, Antoine, 92240 MALAKOFF (FR); RUPPE, Etienne, 75015 PARIS (FR); WOERTHER, Paul Louis, 75011 PARIS (FR); ANGEBAULT, Cécile, 75018 PARIS (FR); DJOSSOU, Félix, 97300 CAYENNE (FR)
(74) Representative: Regimbeau

(57) **Abstract**

The present invention relates to the field of the management of extended-spectrum beta-lactamase (ESBL)-producing *Enterobacteriaceae* colonization. In particular, the present invention relates to methods for assessing the risk to develop intestinal carriage of ESBL-producing *Enterobacteriaceae* (E-ESBL) or for prognosing intestinal carriage of E-ESBL. More specifically, the invention relates to methods comprising a step of determining the abundance of at least one bacterium selected from the list consisting of bacteria from the genus *Desulfovibrio, Oscillospira, Parabacteroides, Coprococcus* and *Prevotella* in the gut microbiota of a subject.

A further object of this invention is to provide a new composition, for mucosal administration, comprising at least one bacterium selected from the list consisting of bacteria from the genus *Desulfovibrio, Oscillospira, Parabacteroides,* and *Coprococcus.* More particularly, this invention relates to such compositions for use in the prevention or treatment of intestinal carriage of E-ESBL in a subject in need thereof.

SEQUENCE LISTING

<110>  Universitat de València
       Fundación para el Fomento de la Investigación Sanitaria y
       Biomédica de la Comunitat Valenciana
       Centro de Investigación Biomédica en Red
       Université Paris Diderot  Paris 7
       ASSISTANCE PUBLIQUE HOPITAUX DE PARIS

<120>  METHODS AND MEANS FOR MANAGING EXTENDED-SPECTRUM BETA-LACTAMASE
       (ESBL)-PRODUCING ENTEROBACTERIACEAE COLONIZATION

<130>  B71309D33774

<160>  2

<170>  PatentIn version 3.5

<210>  1
<211>  20
<212>  DNA
<213>  primer

<400>  1
agagtttgat cmtggctcag                                                   20


<210>  2
<211>  19
<212>  DNA
<213>  primer

<400>  2
tgctgcctcc cgtaggagt                                                    19

## Description

The present invention relates to the field of the management of extended-spectrum beta-lactamase (ESBL)-producing Enterobacteriaceae colonization. In particular, the present invention relates to methods for assessing the risk to develop intestinal carriage of ESBL-producing Enterobacteriaceae (E-ESBL) or for prognosing intestinal carriage of E-ESBL. More specifically, the invention relates to methods comprising a step of determining the abundance of at least one bacterium selected from the list consisting of bacteria from the genus *Desulfovibrio, Oscillospira, Parabacteroides, Coprococcus* and *Prevotella* in the gut microbiota of a subject.

A further object of this invention is to provide a new composition, for mucosal administration, comprising at least one bacterium selected from the list consisting of bacteria from the genus *Desulfovibrio, Oscillospira, Parabacteroides,* and *Coprococcus.* More particularly, this invention relates to such compositions for use in the prevention or treatment of intestinal carriage of E-ESBL in a subject in need thereof.

### BACKGROUND OF THE INVENTION

Antibiotics have revolutionized the treatment of bacterial infections since the first use of penicillin. However, antibiotic overuse has led to an increase in bacterial resistance.

For a long time, the increased resistance to antibiotics has been masked by the continuous discovery and use of new ones. Multi-resistant bacteria were only observed in hospitalized patients, but currently they are increasingly isolated from the community at large. For example, Extended Spectrum Beta-Lactamase producing Enterobacteriaceae (E-ESBL) is a growing public healthcare problem. The incidence of E-ESBL infections in French hospitals has increased from 0.24 to 0.50 per 1000 patient-days, between 2007 and 2011 (Arnaud et al., 2013). This increased incidence can constitute a high threat among population particularly exposed to bacteremia such as patients under chemotherapy or patients in intensive care unit (e.g. anaesthesiology and reanimation). Prior to causing infections, E-ESBL colonize the intestinal microbiota of healthy individuals and can persist in the human gut for years. Hence, intestinal colonization is recognized as the cornerstone of the dissemination of E-ESBL. Epidemiological and individual risk factors for such colonization have been studied extensively but whether it is associated with significant changes in the composition of the rest of the microbiota, as a cause or as a consequence, is still unknown. Known risk factors for intestinal carriage of E-ESBL and infections include international travels, recent hospitalizations, antibiotic use, urinary tract catheters, older age, and diabetes (Soraas et al., 2013).

Hence, there is a need in the art for methods allowing the risk assessment or the prognosis of the gastro-intestinal tract colonization by E-ESBL.

Extended spectrum beta-lactamases (ESBL) carriers are resistant not only to most beta-lactams, but they are also very often co-resistant to the most commonly used antibiotics and can cause very common infections, such as urinary tract infections and pyelonephritis.

Currently, carbapenems are, in general, regarded as the preferred agent for treatment of infections due to E-ESBL. Carbapenems are resistant to ESBL-mediated hydrolysis and exhibit excellent *in vitro* activity against strains of Enterobacteriaceae expressing ESBLs. However, as for all other antibiotics, it recently appeared carbapenem-resistant enterobacteriaceae that are immune to the carbapenem class of antibiotics.

In order to reduce or suppress this intestinal carriage of E-ESBL, a strategy of decolonization of intestinal carriage thereof with oral colistin and neomycin has been evaluated. The regimen used in this study temporarily suppressed E-ESBL carriage, but had no long-term effect (Huttner et al., 2013). Plus, the risk for selecting colistin-resistance as the last step for pan-resistance mitigates its wide-scale deployment.

There is thus a need in the art for means allowing a reduction of the prevalence of E-ESBL, particularly intestinal carriage prevalence, which will not be based on classical antibiotherapies and which will allow a long lasting effect.

The human colon harbours a vast ensemble of microbes which is defined as the microbiota. Whereas stomach and small intestine are relatively poor of bacteria, the gut microbiota in humans is a particularly complex ecosystem with few dominant phyla (Firmicutes, Bacteroidetes, Proteobacteria and Actinobacteria) but show greater microbial diversity at lower taxonomic levels and a high functional redundancy. The gut microbiota seems to be involved in a large number of host beneficial functions such as food processing, growth regulation of the intestinal epithelium and development of the immune system. Moreover, an important function of the intestinal microbiota is to prevent colonisation by exogenous bacteria, including potentially pathogenic microorganisms. This resistance to colonisation is associated with the anaerobic intestinal microbiota, but the precise mechanism involved is still largely unknown. Recently, the presence of the *Barnesiella* genus in the intestinal microbiota was shown to prevent vancomycin-resistant *Enterococcus* domination (Ubeda et al., 2013; Singh et al., 2014).

However, to date, there is no means suitable for the prevention or the treatment of an intestinal colonization by E-ESBL based on other mechanisms than antibiotherapy such as microbial antagonism.

Thus there is also a need in the art for methods allowing the prevention or the treatment of the intestinal carriage of E-ESBL. Such methods should limit the dissemination of E-ESBL among the community and reduce the susceptibility of treated subjects to E-ESBL infection.

### SUMMARY OF THE INVENTION

It is an object of the present invention to provide a method for assessing the risk to develop intestinal carriage of E-ESBL or for prognosing intestinal carriage of E-ESBL.

More particularly, the invention relates to methods comprising a step of determining the abundance of at least one bacterium selected from the list consisting of bacteria from the genus *Desulfovibrio, Oscillospira, Parabacteroides, Coprococcus* and *Prevotella* in the gut microbiota of a subject.

A further object of this invention relates to a mucosal composition comprising at least one bacterium selected from the list consisting of bacteria from the genus *Desulfovibrio, Oscillospira, Parabacteroides,* and *Coprococcus.*

Yet a further object of this invention relates to at least one bacterium selected from the list consisting of bacteria from the genus *Desulfovibrio, Oscillospira, Parabacteroides,* and *Coprococcus,* for use in the prevention or treatment of intestinal carriage of E-ESBL in a subject in need thereof.

### BRIEF DESCRIPTION OF DRAWINGS

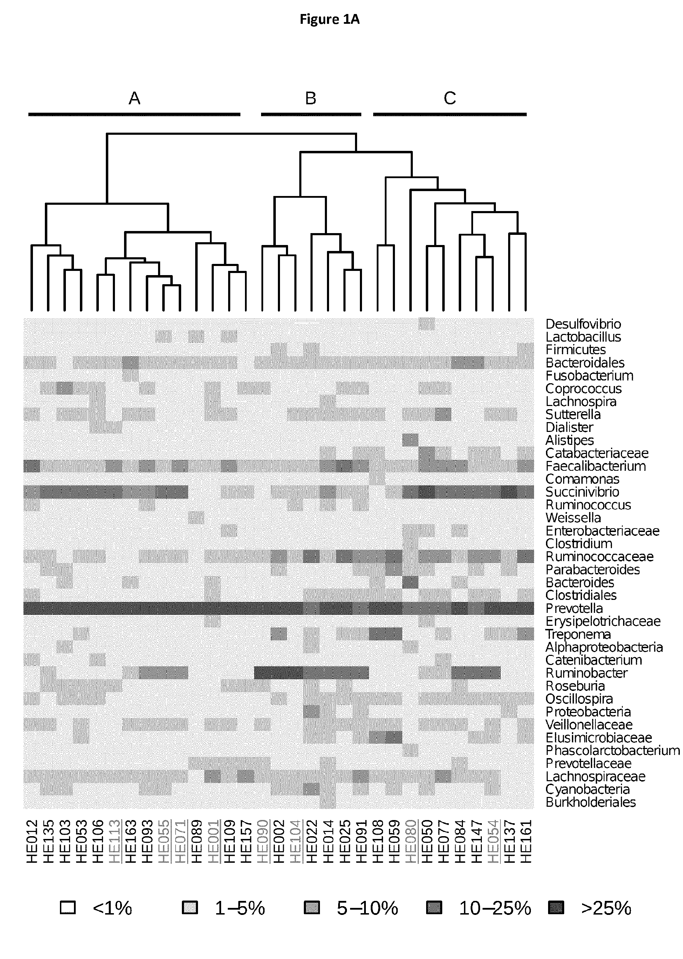

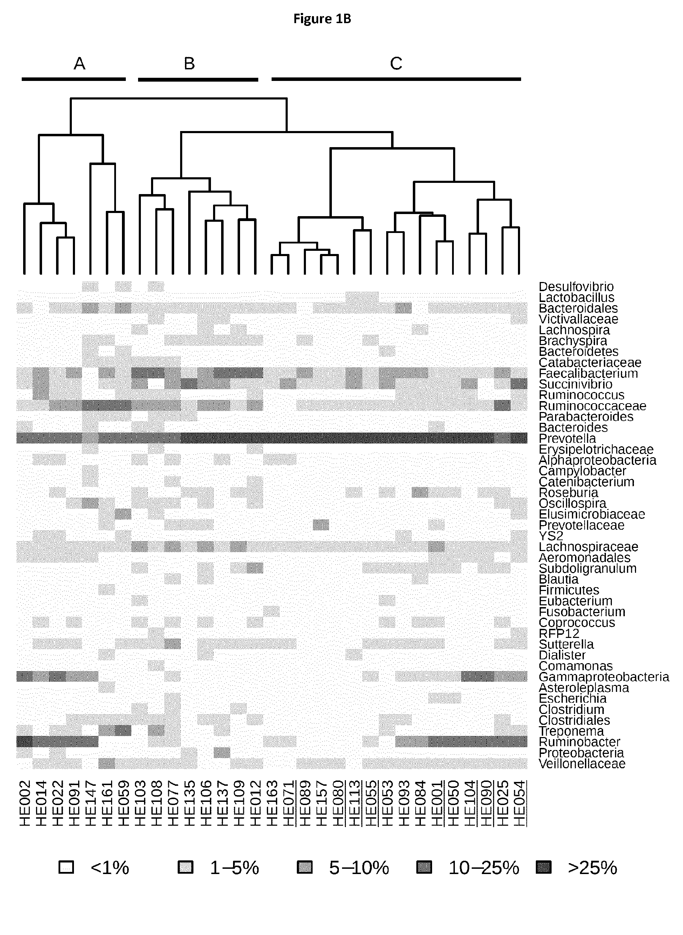

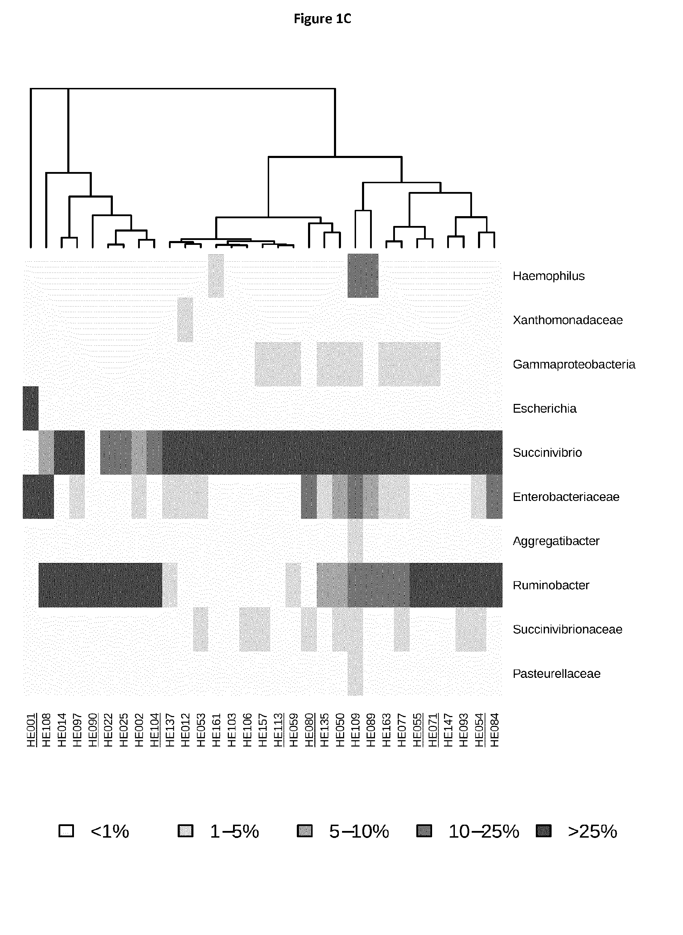

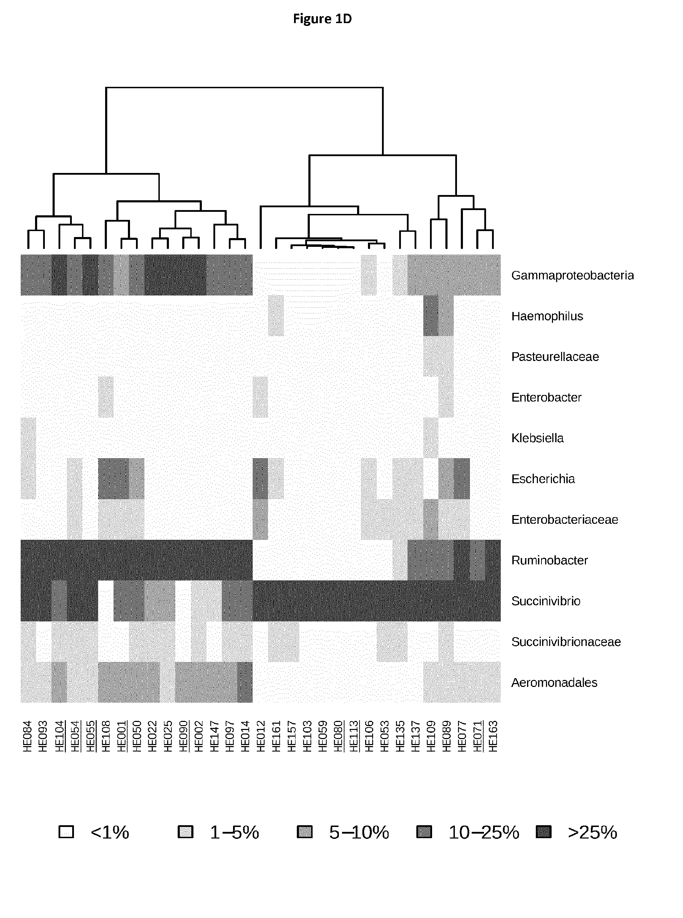

**Figure 1** shows gut microbiota composition through heat maps and clustering based on taxon composition and abundance (Bray-Curtis distance) for (A) the total microbiota (16S rDNA) and (B) the active microbiota (16S rRNA). Analysis has been focused in gamma Proteobacteria from (C) total microbiota and (D) active microbiota. Grey levels in the figures depict the percentage range of sequences assigned to main taxa (abundance >1% in at least one sample). The ESBL carriers are underlined.

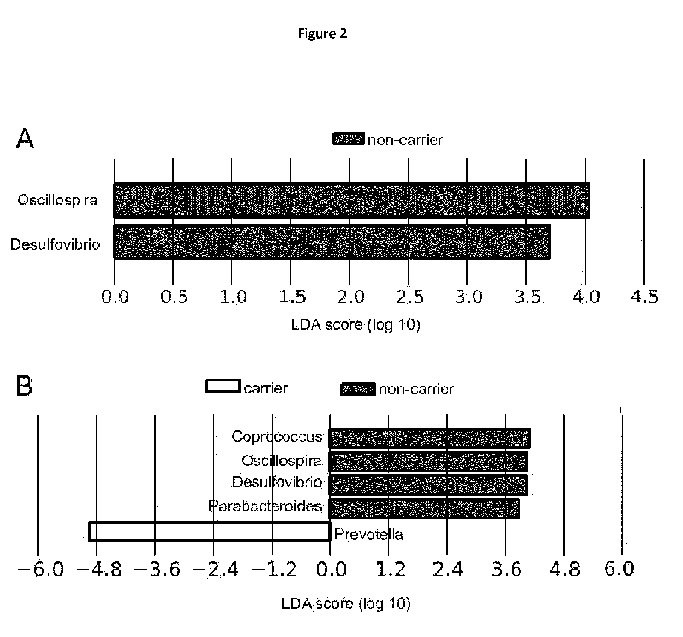

**Figure 2** shows linear discriminant analysis of statistically significant taxa between non-carriers and carriers. (A) Total and active (B) microbiota. The LDA score (log 10) for the most prevalent taxa in the non-carrier samples are represented by the black barplots in the positive scale, whereas the carrier-enriched taxa correspond to the white barplots with LDA negative score.

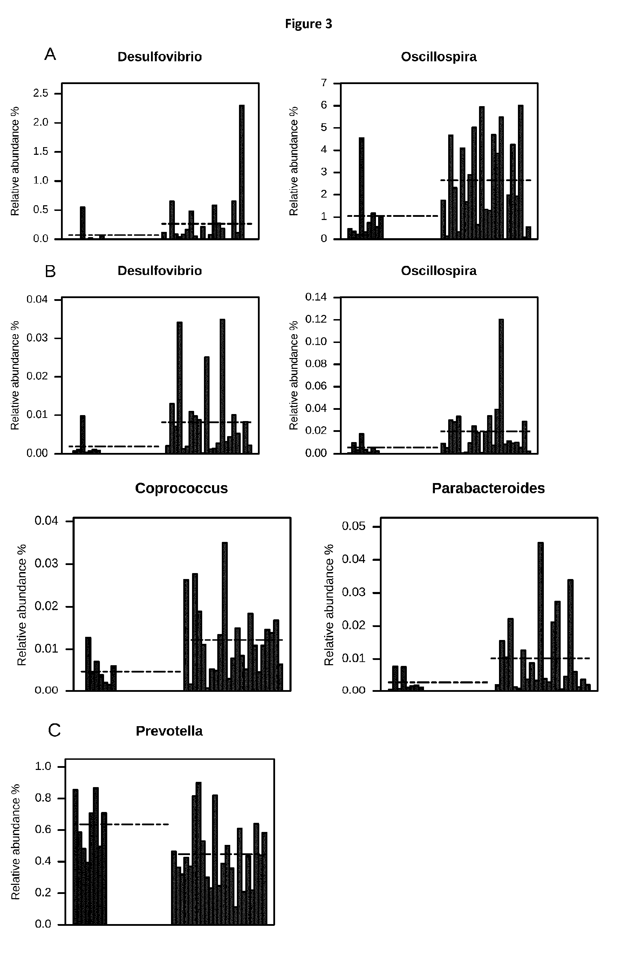

**Figure 3** shows relative abundance of the bacterial biomarkers. (A) Non-carriers' biomarkers in total microbiota, (B) non-carriers' biomarkers in active microbiota and (C) carriers' biomarker in active microbiota. Relative abundance in carriers (left) compared to non-carriers (right), dashed line represents mean value.

### DETAILED DESCRIPTION OF THE INVENTION

The present invention solves the current needs in the art for an efficient management of the spreading of E-ESBL by providing a method for assessing the risk to develop intestinal carriage of E-ESBL or for prognosing intestinal carriage of E-ESBL, along with means and methods for the prevention or the treatment of intestinal carriage of E-ESBL.

The invention stems, *inter alia,* from the unexpected discovery by the inventors, that four genera, *Desulfovibrio, Oscillospira, Parabacteroides* and *Coprococcus* were significantly more abundant in the active microbiota of non-carriers than in that of carriers of E-ESBL. Furthermore, the inventors also showed that carriers presented a significantly greater abundance of *Prevotella* genus in the active microbiota.

From this discovery, the inventors developed a method for assessing the risk to develop intestinal carriage of E-ESBL or for prognosing intestinal carriage of E-ESBL, comprising a step of determining the abundance of at least one bacterium selected from the list consisting of bacteria from the genus *Desulfovibrio, Oscillospira, Parabacteroides, Coprococcus* and *Prevotella* in a sample of the gut microbiota of a subject. Moreover, as it will be shown in this application, *Desulfovibrio, Oscillospira, Parabacteroides* and *Coprococcus* can be used to produce a composition, for mucosal administration, useful for balancing the gut microbial population and thus to prevent or to reduce the intestinal carriage of E-ESBL in a subject in need thereof.

As used herein, the term "prognosing intestinal carriage of E-ESBL" refers to the prediction of the behavior of E-ESBL population among the gut microbiota, either in the absence of, or during or after administration of an appropriate therapy. This may include an estimation of the chances of recovery (absence of detectable E-ESBL in gut microbiota) or of the risks of an augmentation of the abundance of E-ESBL in the gut microbiota for a subject after a time-period.

By "assessing the risk to develop", it is herein meant determining the probability of a subject to have his/her gut microbiota colonized by E-ESBL at a detectable level over a time-period.

As used herein, the term "intestinal carriage of E-ESBL" relates to the presence of E-ESBL in the gastro-intestinal tract which includes intestines, colon and the perianal region. The perianal area includes the perineum and the groin or inguinal region.

The term "sample of feces" as used herein refers to a sample of bodily fluid obtained from feces and that is useful for the purpose of prognosis or risk assessment of a subject of interest.

The term "extended-spectrum beta-lactamase (ESBL)-producing Enterobacteriaceae (E-ESBL)" as used herein designates Enterobacteriaceae secreting extended-spectrum beta-lactamases. Enterobacteriaceae is a large family of Gram-negative bacteria which is well known to the person skilled in the art (Bergey's Manual of Systematic Bacteriology, Volume 2, 2004). ESBLs confer resistance to most beta-lactams (e.g., penicillin, cephalosporin, aztreonam) and are encoded by genes derived through mutations of the ubiquitous plasmid-borne *b*/*a*_{TEM} and *b*/*a*_{SHV} wild-type penicillinase genes or more recently *b*/*a*_{CTX-M} genes.

Enterobacteriaceae herein considered are preferably *Escherichia coli* and *Klebsiella pneumoniae.*

The term "subject" as used herein refers to any animal, including (but not limited to) mammals and aves, which can take benefice from the methods and means of the invention.

As used herein, the term "abundance" refers to the concentration of a group of bacteria in a biological sample. The unit for expressing abundance is depending on the counting method and can include number of nucleic sequence copies and number of clones.

As used herein, the term "relative abundance" refers to the percent of bacteria compared to a reference value in the same biological sample. The reference value can be a wider population, such as whole community or another bacterial group such as Enterobacteriaceae. For example, when molecular methods are used, the relative abundance of *Desulfovibrio* in a sample can be calculated as a percent of total analyzed sequences of the sample.

As used herein, the term "compared abundance" refers to the ratio between the abundance as defined above and a reference value which is obtained from the same biological sample. The reference value used for determining the compared abundance can be the value expected in a healthy or low risk population or the value previously determined of the same subject. For example, the compared abundance refers to ratio between the concentration of a group of bacteria in a sample and the concentration of the same group of bacteria in a non-colonized subject. The compared abundance can also refers to ratio between the concentration of a group of bacteria in a sample and a threshold concentration for this group of bacteria. The threshold concentration of the same group of bacteria corresponds to a concentration which will not be associated with a GIT colonization by E-ESBL. Hence the compared abundance can be calculated according to a previous abundance or a threshold concentration.

As used herein, the term "gut microbiota" refers to the microbial population of the gastro-intestinal tract. More particularly, the term "total gut microbiota" refers to the whole microbial population of the gastro-intestinal tract, whether it is metabolically active or in a quiescent state, as measured for example via the rRNA gene. The term "active gut microbiota" refers to metabolically active bacteria in the gastro-intestinal tract as measured for example via the transcripts of the 16S rRNA gene.

In the context of the invention, the term "treating intestinal carriage of E-ESBL", means reversing or alleviating the intestinal carriage of E-ESBL.

In the context of the invention, the term "preventing intestinal carriage of E-ESBL", means protecting the gastro-intestinal tract from a colonization by E-ESBL. It may be a partial or complete prevention of the gastro-intestinal tract from the colonization by E-ESBL. Thus it includes the capacity to avoid the onset, or to minimize the development of the intestinal carriage of E-ESBL.

As used herein, the term "mucosal composition" refers to various active compositions suitable to reach the intestinal compartment. It is intended to include (but is not limited to) compositions for oral administration, sublingual administration and rectal administration.

As used herein, the term "pharmaceutical carrier" refers to substances that improve the delivery and the effectiveness of an active principle (here the bacteria).

As used herein, the term "prebiotics" refers to compounds that allow specific changes both in the composition and/or in the activity of gastrointestinal microbiota, conferring benefits upon the host well-being and health.

As used herein, a "healthy or low risk population" means a population constituted of subjects who have not previously been diagnosed with E-ESBL infections. In other words, such subjects, if examined by a medical professional, would be characterized as healthy and free of symptoms of E-ESBL infections and preferably free of intestinal carriage of E-ESBL.

In a first aspect, the invention relates to an *in vitro* method for assessing the risk to develop intestinal carriage of E-ESBL or for prognosing intestinal carriage of E-ESBL, comprising a step of determining the abundance of at least one bacterium selected from the list consisting of bacteria from the genus *Desulfovibrio, Oscillospira, Parabacteroides, Coprococcus* and *Prevotella* in a sample from the gut microbiota of a subject.

Indeed, inventors shown that bacteria belonging to *Desulfovibrio, Oscillospira, Parabacteroides* and *Coprococcus* were significantly more abundant in the gut microbiota of non-carriers than in that of carriers of E-ESBL, whereas *Prevotella is* significantly more abundant in the gut microbiota of carriers than in that of non-carriers of E-ESBL.

Hence, determining the abundance of at least one bacterium selected from the list consisting of bacteria from the genus *Desulfovibrio, Oscillospira, Parabacteroides, Coprococcus* and *Prevotella* enables, on the one hand, to assess the risk of a subject to become colonized by E-ESBL and, on the other hand, to prognose the intestinal carriage of E-ESBL of a subject already colonized.

The determination of the abundance can be done by methods well known by the person skilled in the art. For example, techniques for characterizing and quantifying the gut microbiota include the use of nucleic acid analysis.

Nucleic acid analysis includes analysis of, for example, DNA, RNA, mRNA, rRNA, and/or tRNA, and can be accomplished using, for example, pyrosequencing, qPCR, RT-qPCR, clone libraries, DGGE, T-RFLP, ARISA, microarrays, FISH, dot-blot hybridization, next generation sequencing, nanopore sequencing, and any other DNA hybridization methods that will detect a specific sequence.

In one non-limiting example, Next Generation Sequencing (NGS) provides for high-capacity, low-cost sequencing.

Advantageously, nucleic acid analysis can be based on the sequence of the 16S rRNA gene and/or the corresponding transcript sequences. 16S rRNA is a component of the 30S small subunit of prokaryotic ribosomes. The encoding genes are referred to as 16S rDNA and are used in reconstructing phylogenies as they are highly conserved between different species of bacteria. Primers used for the amplification of such sequences before sequencing are well-known by the person skilled in the art. List of such primers can be found in Baker et al. 2003.

In one non-limiting example, primers listed in table I can be used for nucleic acid analysis based on the 16S rRNA gene.

**Table I**

| Primer name | Sequence | Ref |
|---|---|---|
| E8F | 5'-AGAGTTTGATCMTGGCTCAG-3' | SEQ ID n°1 |
| 357R | 5'- TGCTGCCTCCCGTAGGAGT-3' | SEQ ID n°2 |

These characterizations can be combined with statistical analysis to determine the constituents of the gut microbiota. The present disclosure uses different statistical tests and correction methods for multiple testing that strengthen the interpretation of the obtained data. Bioinformatics helps to achieve an efficient definition of the characteristics and distributions of intestinal microbiota between subjects. For example, an appropriate method can be found in examples below.

The Inventors showed that determining the abundance of at least one bacterium belonging to one of five genera can be relevant for the management of E-ESBL colonization of the gastro-intestinal tract (GIT). Those five genera correspond to anaerobic bacteria described below.

The genus *Desulfovibrio* comprises Gram negative rod-shaped bacteria identified as anaerobically growing, non-spore forming motile organisms and about 0.7 um in cell diameter. *Desulfovibrio* is known as a sulfate reducing bacterium. To date, the genome of two strains have already been sequenced: *Desulfovibrio desulfuricans* G20 and *Desulfovibrio vulgaris* subsp. *vulgaris* str. Hildenborough. Both of the completely sequenced genomes showed *Desulfovibrio* to have one chromosome measuring over 3 Mbp in length.

The *Oscillospira* genus, belonging to the order of Clostridiales, family Ruminococcaceae, comprises large bacterium (3-6 x 10-40 µm) often observed in the rumen contents of sheep and cattle as well as the alimentary tract of other herbivorous animals or humans. Although no pure culture were reported, it is defined as comprising Gram-positive bacteria affiliated with the low G+C subclass (Firmicutes).

The genus *Parabacteroides* comprises rod-shaped, 0.8-1.6×1.2-12 µm in size, Gram-negative bacterium, obligately anaerobic, non-spore-forming and non-motile. It can be found in the human GIT microbiota. This genus has been created recently from members of the *Bacteroides* genus.

The genus *Coprococcus* comprises Gram-positive cocci which are strictly anaerobic and chemoorganotrophs. All described species have been isolated from human intestinal contents or feces. They can be grown in pure culture and fermentable carbohydrates are either required are highly stimulatory for growth.

The genus *Prevotella* comprises anaerobic, non spore forming, Gram-negative non motile rods. They are common inhabitant of human intestines. As for *Parabacteroides,* this genus has been recently created from members of the *Bacteroides* genus.

A genus generally contains several bacterial species which can in turn contain several subspecies, varieties and ultimately strains. The abundance is determined for at least one bacterium from the selected genus. Hence, according to the invention, when determining the abundance of at least one bacterium from the selected genus, the person skilled in the art will understand that it is sufficient to refer to a group of bacteria belonging to the analyzed genus. This group of bacteria can contain only one strain of the analyzed genus but it can also contain several varieties or species provided that all the bacteria of this group belong to the analyzed genus.

In particular, the abundance is determined for all the bacteria belonging to specie of the selected genus. For example, the abundance is determined for all the bacteria belonging to the *Desulfovibrio piger* species.

Yet in particular, the abundance is determined for all the bacteria of the selected genus. For example, the abundance is determined for all the bacteria belonging to the *Desulfovibrio* genus.

Preferably, the method of the invention comprises a step of determining the abundance of at least one bacterium of at least two genera selected from the list consisting of the genus *Desulfovibrio, Parabacteroides, Prevotella, Oscillospira* and *Coprococcus.*

In another preferred embodiment, the method comprises a step of determining the abundance of at least one bacterium of each of the genus *Desulfovibrio, Parabacteroides, Prevotella, Oscillospira* and *Coprococcus.*

The analysis of several genera allows an enhancement of the sensitivity of the prognosis/risk assessment method.

In a more preferred embodiment, the method comprises a step of determining the abundance of at least one bacterium of the genus *Desulfovibrio and* at least one bacterium *Prevotella.*

In an embodiment, it is the abundance of at least one bacterium of the total gut microbiota of said subject which is determined in a sample. The total gut microbiota is preferably determined by DNA based molecular analysis.

In a preferred embodiment, it is the abundance of at least one bacterium of the active gut microbiota of said subject which is determined in a sample. The active gut microbiota is preferably determined by RNA based molecular analysis. In this embodiment, the method of the invention comprises the step of retro-transcribing RNA into single-stranded cDNA and synthesizing double-stranded cDNAfrom the latter.

According to the invention, a high abundance of bacteria belonging to genus *Desulfovibrio, Oscillospira, Parabacteroides,* and *Coprococcus* is indicative of a low susceptibility to intestinal carriage of E-ESBL.

Instead, according to the invention, a high abundance of bacteria belonging to the genus *Prevotella* is indicative of a higher susceptibility to intestinal carriage of E-ESBL.

According to the invention, it can be considered that a significant shift on the abundance or relative abundance is a 10 %, preferably a 30 % and more preferably a 50 % shift. Hence, a high compared abundance can be an abundance or relative abundance 10 % higher than the previous abundance, the threshold concentration or the ratio calculated with reference bacterial group in a healthy or low risk population.

In a preferred embodiment, the abundance of at least one bacterium is determined from a sample of feces from said subject. Preferred test samples include stools and can be obtained for example through the use of rectal swabs or anal swabs. In addition, one of skill in the art will understand that some test samples would be more readily analyzed following a fractionation or purification procedure.

In the context of the invention, the subject is an animal. It can be animals belonging to the mammal's class such as primates, or bovidaes, or animals belonging to the aves class such as Gallinaes. In particular, the subject according to the invention is selected from the group consisting of cattle, poultry and human. Preferably, the subject is a bovidae, a suidae, a phasianidae or a human. More preferably, the subject is a human.

More preferably, methods according to the invention can be used on human which are at risk of developing an intestinal carriage of E-ESBL such as international travelers, patients in intensive care units or patient under chemotherapy.

Methods according to the invention will identify subjects which are at risk of developing an intestinal carriage of E-ESBL because their gut microbiota is not prepared to prevent colonization. Hence, they will permit to identify subjects which will take benefit from a treatment to reduce the risk of developing an intestinal carriage of E-ESBL or to improve the prognosis of the intestinal carriage of E-ESBL.

The methods of the invention can further comprise a step of administering to the subject a treatment to reduce the risk of developing an intestinal carriage of E-ESBL or to improve the prognosis of the intestinal carriage of E-ESBL.

Another object of the invention is a mucosal composition comprising at least one bacterium selected from the list consisting of bacteria from the genus *Desulfovibrio, Parabacteroides, Oscillospira* and *Coprococcus.*

In an embodiment, the invention relates to a mucosal composition comprising at least one bacterium selected from the list consisting of bacteria from the genus *Desulfovibrio, Parabacteroides,* and *Coprococcus.*

Preferably, the invention relates to a mucosal composition comprising at least one bacterium from each of the genus *Desulfovibrio* and *Coprococcus.* Yet preferably, the invention relates to a mucosal composition comprising at least one bacterium from each of the genus *Desulfovibrio, Parabacteroides,* and *Coprococcus.*

Mucosal compositions can contain one or more appropriate pharmaceutical vehicles or carriers which may be used in order to ensure a suitable mucosal delivery of each to the expected site (e.g., a mucosal surface).

A mucosal delivery is typically selected from nasal, oral, sub-lingual, tracheal, pharyngeal, bronchial, esophageal, gastric, duodenal, intestinal, rectal, preputial and vaginal deliveries. A mucosal delivery is a delivery to a mucosal surface, such as nasal, oral, sub-lingual, tracheal, bronchial, pharyngeal, esophageal, gastric, and mucosae of the duodenum, small and large intestines, including the rectum, as well as preputial and vaginal mucosae. Preferably, the mucosal surface refers to digestive mucosa. Yet preferably, the mucosal delivery is an oral delivery.

Preferably, the mucosal composition according to the invention is an orally administrable composition. Orally administrable composition can be any dosage form which can be administered orally, such as, but not limited to: a capsule, a tablet, a powder that can be dispersed in a beverage, a liquid such as a solution, suspension, or emulsion, a soft gel/chew capsule, a chewable bar or other convenient dosage form such as oral liquid in a capsule, as known in the art.

The mucosal composition may further comprise at least one absorption agent. "Absorption agents" are well known from the one of skill in the art. As examples, one can cite surfactants such as polyoxyethylene derivatives of fatty acid partial esters of sorbitol anhydrides (e.g., Tween® 80, Polyoxyl 40 Stearate, Polyoxyethylene 50 Stearate, polyoxyethylene-9-lauryl ether and Octoxynol), bile salts such as sodium glycocholate, mixed micelles, enamines, nitric oxide donors (eg., S-nitroso-N-acetyl-DL-penicillamine, NOR1, NOR4-which are preferably co-administered with an NO scavenger such as carboxy-PITO or doclofenac sodium), sodium salicylate, glycerol esters of acetoacetic acid (eg., glyceryl-1,3-diacetoacetate or 1,2-isopropylideneglycerine-3-acetoacetate), cyclodextrin or beta-cyclodextrin derivatives (eg., 2-hydroxypropyl-beta-cyclodextrin and heptakis(2,6-di-O-methyl-beta-cyclodextrin)), medium-chain fatty acid such as mono- and diglycerides (eg., sodium caprate-extracts of coconut oil, Capmul), or triglycerides (eg., amylodextrin, Estaram 299, Miglyol 810), polymers such as carboxymethylcellulose, carbopol, polycarbophil, tragacanth and sodium alginate.

In an embodiment, the mucosal composition comprises a prebiotic. Preferred prebiotics according to the present invention are prebiotics which allow improving gut colonization by bacteria belonging to the genus *Desulfovibrio, Parabacteroides, Oscillospira* or *Coprococcus.* Prebiotics are preferably selected from non digestible oligosaccharides, such as fructooligosaccharides (FOS), galactooligosaccharides (GOS), lactulose, and inulin. They cannot be adsorbed until they reach colon, where they can be fermented by a specific microbiota into small chain fatty acid (SCFA) and lactate.

Mucosal compositions comprising at least one bacterium selected from the list consisting of bacteria from the genus *Desulfovibrio, Parabacteroides* and *Coprococcus* can be prepared by methods well known of the person skilled in the art.

The time and dose for administering each of the bacteria will be easily adapted by the skilled artisan.

In another object, the invention relates to at least one bacterium selected from the list consisting of bacteria from the genus *Desulfovibrio, Parabacteroides, Oscillospira* and *Coprococcus,* or a composition comprising same, for use in the prevention or treatment of intestinal carriage of E-ESBL in a subject in need thereof.

Preferably, the intestinal carriage of E-ESBL in a subject is monitored along the treatment. Intestinal carriage of E-ESBL can be followed by methods well known to the person skill in the art such as classical culture assays with antibiotics or nucleic acid analysis based on 16S rRNA gene and ESBL DNA. For example, Enterobacteriaceae isolates can be screened for possible ESBL production by use of the double disk diffusion method.

Preferably, according to the invention, there is intestinal carriage of ESBL-E when the presence of at least one E-ESBL is detected by technical means available to the person skilled in the art.

The subject in need thereof can be treated for at least one day, preferably at least three days, more preferably one week. In a preferred embodiment, the treatment begins two weeks before the beginning of potential risk of colonization. The treatment should be as long as the subject is exposed to the risk for instance for the travelers the length of the stay in endemic areas.

Advantageously, the assessment of intestinal carriage of E-ESBL in a subject along the treatment can allow the adaptation of the treatment and particularly the dosage regime.

The treatment according to the invention can be coupled with a method of prognosis of intestinal carriage of E-ESBL according to the invention.

Preferably, a subject in need thereof is an animal. It can be animals belonging to the mammal's class such as primates, suidaes or bovidaes, or animals belonging to the aves class such as Gallinaes. In a preferred embodiment, the subject according to the invention is selected from the group consisting in suidaes, cattle, sheep, poultry and human. In a preferred embodiment, the subject is a human.

Preferably, the subject in need thereof is a human which are at risk of developing an intestinal carriage of E-ESBL such as international travelers, patients in intensive care units or patient under chemotherapy.

In another aspect, the invention relates to a method for the treatment of intestinal carriage of E-ESBL comprising a step of administering a mucosal composition comprising at least one bacterium selected from the list consisting of bacteria from the genus *Desulfovibrio, Oscillospira, Parabacteroides,* and *Coprococcus.*

All the aspects of the present invention preferably involve all the features (including any illustrative, advantageous, or preferred features) that have been described above with respect to the first aspect of the invention. This means that all the herein-described features of the first aspect can apply *mutatis mutandis* to anyone of the aspects of the present invention.

### EXAMPLES

### I. Method

### I.1. Study subjects and sampling

The volunteers enrolled in the study were healthy adult Wayampi Amerindians from the village of Trois-Sauts in French Guyana. This is a genetically homogenous population of -500, still living mostly in a traditional manner, including alimentation which is provided by hunting, fishing and raising crops. However, a resident paramedic ensures fully free access to allopathic medicine and records all healthcare deliveries, including antibiotics. During the last sampling campaign, which took place in October 2010, 151 individuals were tested for E-ESBL intestinal carriage, as follows. Fresh fecal samples were inoculated immediately onto Drigalski agar slants in screw-cup tubes and sent to mainland France within 2 weeks, at room temperature. There, the whole culture from each tube was suspended in 1.5 ml of brain heart infusion broth with 10% glycerol and stored at -80°C. Upon harvesting, 100-µl aliquots of each broth were cultured on agar plates selective for extended-spectrum cephalosporin-resistant strains containing 1.5 mg/liter cefotaxime and 2 mg/liter ceftazidime (ESBL; AES Chemunex, Bruz, France). All Enterobacteriaceae with different morphotypes that grew on these plates were identified at the species level using a Maldi Biotyper system (Bruker Daltonics, Bremen, Germany). Their antimicrobial susceptibility was determined using the disk diffusion method, as previously described (http://www.sfm-microbiologie.org/). In addition, a freshly passed (less than 2 hrs) faecal sample of about 5 g was extemporaneously diluted 1:10 in RNAlater (Applied Biosystems, Villebon-sur-Yvette, France), thoroughly mixed and immediately frozen at -20°C before being taken to the laboratory, without defrosting, and stored at -80°C until harvesting.

Eight (5.3%) of the 151 individuals were found to be E-ESBL carriers. Among them, 4 were carrying a CTX-M-1 type ESBL, two were carrying a CTX-M-8 ESBL and one was carrying a CTX-M-2 ESBL. The last carrier was carrying both CTX-M-2 and CTX-M-8 ESBLs (AA). In order to identify possible specificities in the microbiota of the carriers, the microbiota of 23 non-carriers (3 per carrier except in one case) who were chosen to match carriers as closely as possible in terms of age, gender and antibiotic consumption was analysed within the year preceding sampling.

Signed informed consent was obtained from all subjects and the study protocol was approved by the *ad hoc* Ethical Committee (Comité de Protection des Personnes Sud-Ouest et Outre Mer III, 2010-A00682-37).

### 1.2. Nucleic acids purification

The faecal samples were defrosted and homogenized and 5 ml of each were diluted with 5 ml of phosphate buffered saline (PBS) (containing, per litre, 8 g of NaCl, 0.2 g of KCI, 1.44 g of Na2HPO4, and 0.24 g of KH2PO4 [pH 7.2]). Then, they were centrifuged at 2000 rpm at 4°C for 2 minutes to remove faecal debris. The supernatant was centrifuged at 13000 rpm for 5 min to pellet the cells. Total DNA and RNA were extracted from pelleted cells using AllPrep DNA/RNA/Protein Mini Kit (QIAGEN, Hilden, Germany) following the manufacturer's instructions. The DNA and RNA extractions were checked by running a standard agarose gel electrophoresis and were quantified with Nanodrop-1000 Spectrophotometer (Thermo Scientific, Wilmington city, USA).

### 1.3. Amplification of the 16S rRNA gene

For each sample a region of the 16S rRNA gene was amplified by polymerase chain reaction (PCR) with the universal primers E8F (SEQ ID n°1) and 357R (SEQ ID n°2) using the sample-specific Multiplex Identifier (MID) for pyrosequencing. The amplified region comprises hyper-variable regions V1 and V2. For each sample a 50µl PCR mix was prepared containing: 5µl of Buffer Taq (10X) with 20mM MgCl₂, 2µl of dNTPs (10mM), 1µl of each primer (10mM), 0.4µl of Taq Fast start polymerase (5u/µl), 39.6µl of nuclease-free water and 1µl of DNA template. PCR was run under the following conditions: 95° for 2 min followed by 25 cycles of 95° for 30 s, 52° for 1 min and 72° for 1 min and a final extension step at 72° for 10 min. The amplification process was checked by electrophoresis in agarose gel (1.4%). PCR products were purified using NucleoFast® 96 PCR Clean-Up Kit (Macherey-Nagel) and quantified with Nanodrop-1000 Spectrophotometer (Thermo Scientific) and with the QuantiT PicoGreen dsDNA Assay Kit (Invitrogen). The pooled PCR products were directly pyrosequenced.

### 1.4. cDNA synthesis

Total RNA was retro-transcribed into single-stranded cDNA using the High-Capacity cDNA Reverse Transcription Kit (Ambion, Carlsbad, California USA) as recommended by the manufacturer, and double-stranded cDNA (ds-cDNA) was then synthesized following standard procedures. The products were quantified using the QuantiT PicoGreen dsDNA Assay Kit (Invitrogen, Carlsbad, California USA). A standard agarose gel electrophoresis was run to check the integrity of double-stranded cDNA. Total ds-cDNAs obtained from each faecal sample were directly sequenced.

### 1.5. Pyrosequencing and Sequence analysis

Pyrosequencing was performed using a Roche GS FLX sequencer and Titanium chemistry in the Centre FISABIO-Salud Pública (Valencia Spain). All sequences were deposited in the European Bioinformatics Institute (http://www.ebi.ac.uk/) database, under accession number ERP001842.

16S rRNA amplicons (hereafter 16S rDNA) with low quality score (<20) and short read lengths (<100 nt) were removed. Potential chimeras were also removed from the remaining sequences using the Chimera-Slayer package of the Mothur software.

To select the 16S rRNA reads from the total RNA sequences, the reads were compared against the Small Subunit rRNA Reference Database (SSUrdb)(Urich et al., 2008) using BLASTN with an e-value of 10⁻¹⁶.

Taxonomic information of the transcripts of 16S rRNA gene (hereafter 16S rRNA, active bacteria) and 16S rDNA (total bacteria) sequences were obtained by comparison with the Ribosomal Database Project-II (RDP) using pick_otus_through_otu_table.py pipe line available in QIIME v1.4.0 software. Operational Taxonomic Units (OTUs) were created by Uclust applying cluster criteria of 97% similarity. The most representative sequences for each OTU were then compared against the QIIME cluster version of the green genes database (database gg_97_otus_4feb2011_aligned.fasta). The annotation was accepted if the bootstrap confidence estimation value was over 0.8, stopping the assignation at the last well identified phylogenetic level. Representative sequences were aligned using the Pynast against the clustered version of the green genes database (database gg_97_otus_4feb2011_aligned.fasta). The aligned sequences were used to reconstruct the phylogenetic tree using the FastTree software.

The Chao1 richness estimator was computed to assess the expected number of bacterial taxa in the samples and the Shannon index of biodiversity to measure the level of homogeneity in the microbiota composition.

### 1.6. Epidemiological data and statistical methods

Each of the eight ESBL carriers was matched with three non-carriers, chosen among the 143 possible ones (the 151 sampled individuals, except the 8 carriers), as close as possible in terms of gender, age and antibiotic exposure. Epidemiological characteristics of carriers and non-carriers were compared by univariate analysis of variance using a Fisher exact test.

Concerning the microbiota, the values were expressed as mean ± standard deviation (SD). Differences in Shannon diversity index and Chao1 estimator were analyzed using the Mann-Witney test30 with a significance level of < 0.05. Heat maps of taxa abundance and composition and clustering based on the Bray-Curtis distance were analyzed using the free statistical package R (http://cran.r-project.or/). Biodiversity between groups of samples was evaluated using the pipeline jackknifed_beta_diversity.py available in QIIME v1.4.0. The rooting tree method was performed by adding two Arqueal 16S sequences (AB294259 and AB294260) to the phylogenetic tree, the rest of the settings being left as default. Weighted UniFrac was chosen to estimate beta diversity because its components better explain the genetic variability between samples.

The linear discriminant analysis (LDA) effect size (LEfSe) algorithm from the Galaxy software package was used to identify specific taxa as biomarkers for carriers and non-carriers. LEfSe first identifies the significant differences in taxa composition between groups (carriers and non-carriers) by applying the Kruskal-Wallis test. Then, the Wilcoxon test was used to check all pairwise comparisons considering that for each group there were individuals treated or not with antibiotics. A cut-off α value <0.05 was fixed for both type of tests. The bacterial taxa with significant differences between samples were used to build the LDA model and to estimate its effect as a discriminant feature among them. The threshold used to consider a discriminative feature for the logarithmic LDA score was set to > 2.0.

**Table 2. Epidemiological characteristics of carriers and non-carriers in the present study.**

| Univariate analysis was performed by an analysis of variance using a Fisher exact test. Differences in Shannon diversity index and Chao1 richness estimator were analyzed using the Mann-Witney test. P is probability at α = 0.05. | | | |
|---|---|---|---|
| | **Carriers N=8** | **Non-carriers N=23** | ***P*** |
| **Sociodemographic data** | | | |
| Average age, years [range] | 46.1 [33-65] | 41.4 [24-67] | 0.32 |
| Sex ratio (F/M) | 3 | 1.1 | 0.41 |
| Marital status / Stable relationship | 7 | 20 | 0.95 |
| Median number of children | 6 | 5 | 0.30 |
| Median number of close relatives | 6 | 6 | 0.82 |

| **Household** | | | |
|---|---|---|---|
| Median number of inhabitants | 7 | 7 | 0.59 |
| Animals in the household | 6 | 17 | 1.00 |

| **Lifestyle** | | | |
|---|---|---|---|
| Drinking water | | | |
| River | 3 | 9 | 0.68 |
| Cove | 2 | 10 | 1.00 |
| Tap water | 6 | 14 | 0.69 |
| Duty | | | |
| Hunting | 2 | 11 | 0.41 |
| Fishing | 7 | 19 | 1.00 |
| Manioc culture | 7 | 20 | 1.00 |
| Cooking | 7 | 18 | 1.00 |
| Cachiri preparing | 6 | 13 | 0.43 |
| Pirogue driver | 4 | 14 | 0.69 |
| Health worker | 1 | 1 | 0.46 |
| Baby-sitting children ≤ 5y | 8 | 16 | 0.15 |
| Travel outside *Trois-Sauts* during past year | 6 | 20 | 0.58 |
| Having a child at school outside *Trois-Sauts* | 3 | 6 | 0.66 |

| **Medical history during past year** | | | |
|---|---|---|---|
| Hospitalisation | 0 | 2 | 1.00 |
| Serious medical event | 1 | 1 | 0.46 |
| H1N1 vaccination | 2 | 5 | 1.00 |
| Antibiotic use | 3 | 3 | 0.16 |
| Antibiotic use among relatives | 6 | 21 | 0.27 |

| **Co-colonisation** | | | |
|---|---|---|---|
| Intestinal colonisation with *Candida albicans* | 2 | 3 | 0.58 |
| Intestinal colonisation with *Candida krusei* | 7 | 17 | 0.64 |
| Intestinal colonisation with *Saccharomyces cerevisiae* | 8 | 18 | 0.29 |

| **Biodiversity of total microbiota** | | | |
|---|---|---|---|
| Shanon index (mean ± SD) | 1.91 ± 0.47 | 2.16 ± 0.49 | 0.10 |
| Chao1 estimator (mean ± SD) | 44.7 ± 9.94 | 50.68 ± 9.63 | 0.24 |

| **Biodiversity of active microbiota** | | | |
|---|---|---|---|
| Shanon index (mean ± SD) | 1.91 ± 0.65 | 2.5 ± 0.57 | 0.02 |
| Chao1 estimator (mean ± SD) | 86.26 ± 5.39 | 83.2 ± 121.71 | 0.84 |

### II. Results

Here, total and active gut microbiota in relation to E-ESBL carriage in an Amerindian population were explored and compared using DNA and RNA high-throughput sequencing methods. The gut microbiota of carriers and non-carriers did not differ significantly when compared for any of the characteristics, including antibiotic exposure (3/8 in carriers vs. 3/23 in non-carriers, p=0.16) (Table 2). The three carriers received metronidazole, cotrimoxazole and amoxicillin, respectively, whereas the three non-carriers received coamoxiclav or metronidazole (one and two subjects, respectively). It is worth noticing that all courses of antibiotic were administered at least six weeks before sampling.

An average of 2,500 16S rDNA sequences per sample was obtained, with the phyla Bacteroidetes (45%) and Firmicutes (40%) being the most abundant, followed by Proteobacteria (12%), Spirochaetes (1.8%) and Tenericutes (0.27%). Actinobacteria were present in only four individuals and with few sequences. Figure 1a shows the heat map based on taxon abundance and sample composition. Three major clusters (named A, B and C) were obtained and the eight E-ESBL carriers were scattered among them (4, 2 and 2 in cluster A, B and C, respectively). In almost all the samples, *Prevotella* genus (Bacteroidetes phylum) was the most abundant taxon. Most of the Firmicutes sequences belonged to the families Lachnospiraceae, Ruminococcaceae and Clostridiaceae. Focusing only on gamma-Proteobacteria, *Succinivibrio* and *Ruminobacter* genera, both members of the family Succinivibrionaceae, were the main taxa, whereas the Enterobacteriaceae family (to which *E. coli* belongs) was less abundant, even in the eight E-ESBL carriers (Fig. 1c). Neither Shannon biodiversity index nor Chao1 richness estimator differed significantly between carriers and non-carriers (1.91 ± 0.47 vs. 2.16 ± 0.49; p = 0.104 and 44.7 ± 0.94 vs. 50.68 ± 9.63; p = 0.24, respectively) (Table 2). The weighted UniFrac analysis was not able to separate carriers and non-carriers either. In addition, no cluster was obtained on the bases of the type of CTX-M genes carried. The results of LEfSe analysis revealed, however, that two biomarkers, *Desulfovibrio and Oscillospira,* were significantly more abundant in the non-carriers than in the carriers (0.264 ± 0.492 *vs.* 0.074 ± 0.181, p = 0.047 and 2.652 ± 2.025 vs. 1.048 ± 1.349, p = 0.035, respectively) (Fig. 2a and Fig. 3a). Both genera are low-abundance taxa but unlike *Desulfovibrio,* detected only in two carriers, *Oscillospira* were present in all the samples and were, on average, 2.5 times more abundant in non-carriers than in carriers (Fig. 3a). By contrast, no significant biomarkers were found in carriers vs. non-carriers (Fig. 2a).

Direct sequencing of the cDNA products gave a mean of 17,972 16S rRNA sequences per sample with an average length of 313 bp. The heat map (Fig. 1b) showed that samples also grouped into three mayor clusters, but in a different way from that observed in the total microbiota. In fact, all eight carriers were grouped in cluster C whereas the non-carriers were evenly distributed among clusters. Similar to the total microbiota, the active microbiota was also characterized by a high abundance of members of the genus *Prevotella* ranging from 25% to 80% of the total bacterial taxa detected. There was also a low abundance of enterobacteria, with the genus *Escherichia,* ranging from 0% to 2% of total sequences (Fig. 1d). When the active microbiota composition was compared in carriers vs. non-carriers, it was found significant differences with the Shannon index (1.91 ± 0.65 vs. 2.50 ± 0.57, p = 0.018) but not with the Chao1 estimator (86.26 ± 25.39 vs. 83.21 ± 21.71, p = 0.84) (Table 2), indicating that the expressed bacteria of ESBL carriers was as taxa rich as those of non-carriers, but less homogeneous.

When the overall composition of the active microbiota was analyzed using the UniFrac metric, the ESBL carriers clustered together, indicating that, contrary to the obtained with total bacteria, the bacterial composition was more similar between them than between the other samples. By contrast, as it occurred with the total bacteria, the active ones did not cluster according to the CTX-M allele type of carriers.

Using the biomarker discovery software LEfSe, it was identified several discriminating genera between carriers and non-carriers, as shown by the LDA score in Figure. 2b. Non-carriers had 4.5 times more *Desulfovibrio* (0.0081 ± 0.01 *vs.* 0.0017 ± 0.003, p = 0.01), 3.7 times more *Oscillospira* (0.0197 ± 0.025 *vs.* 0.0053 ± 0.005, p = 0.03), 3.5 times more *Parabacteroides* (0.0102 ± 0.012 *vs.* 0.0029 ± 0.003, p = 0.04), and 2.5 times more *Coprococcus* (0.0122 ± 0.008 *vs.* 0.0048 ± 0.004, p = 0.01) than carriers (Fig. 3b). Carriers, on the other hand, had 1.4 times more abundant *Prevotella* than non-carriers (0.63 ± 0.17 vs. 0.44 ± 0.20, p = 0.02) (Fig. 3c).

Two anaerobic taxa, belonging to the genus *Desulfovibrio* (Desulfovibrionaceae) and *Oscillospira* (Ruminococcaceae), appeared significantly more abundant in the total microbiota of non-carriers than in carriers. When the active microbiota was considered, two additional genera *(Parabacteroides* and *Coprococcus)* were also significantly more abundant in non-carriers. By contrast, there was only one genus *(Prevotella)* that was more abundant in the active microbiota of carriers than in non-carriers.

Two characteristics of the population studied make these results particularly meaningful. First, the study was performed on subjects from an isolated community, in which antibiotic exposure of each individual in the year preceding sampling was known exactly. This exposure was low in both carriers and non-carriers and not significantly different from each other. Thus, differences in antibiotic exposure were unlikely be the reason for the differences observed. Second, the subjects from this community live a very traditional lifestyle and eat exclusively what they cultivate, fish or hunt. Their diet is not very varied and they all have approximately the same one.

The microbiota of the Wayampi was quite homogenous, and the microbiota of ESBL carriers in terms of taxa richness was less homogeneous than that of non-carriers. Non-carriers had differential anaerobic bacteria in both total and active microbiota that could play a role in colonization resistance, while *Prevotella* appeared as a biomarker for the active microbiota of carriers.

### REFERENCES

I Arnaud, O Bajolet, X Bertrand, H Blanchard, E Caillat-Vallet, C Dumartin, M Eveillard, T Fosse, N Garreau, O Hoff, N Marty, S Maugat, E Reyreaud, A Savey, H Sénéchal, L Simon, E Sousa, D Trystram, B Coignard, V Jarlier, P Astagneau. Regional trends in enterobacteriaceae extended-spectrum betalactamase-producing (ESBLE) and methicillin-resistant staphylococcus aureus (MRSA) between 2007 and 2011. Antimicrobial Resistance and Infection Control 2013, 2(Suppl 1):O34
GC Baker, JJ Smith, DA Cowan.. Review and re-analysis of domain-specific 16S primers. Journal of Microbiological Methods 2003 55:541-555.
B Huttner, T Haustein, I Uçkay, G Renzi, A Stewardson, D Schaerrer, A Agostinho, A Andremont, J Schrenzel, D Pittet, S Harbarth. Decolonization of intestinal carriage of extended-spectrum β-lactamase-producing Enterobacteriaceae with oral colistin and neomycin: a randomized, double-blind, placebo-controlled trial. J Antimicrob Chemother. 2013 Oct;68(10):2375-82.
A Søraas, A Sundsfjord, I Sandven, C Brunborg, P.A. Jenum. Risk Factors for Community-Acquired Urinary Tract Infections Caused by ESBL-Producing Enterobacteriaceae - A Case-Control Study in a Low Prevalence Country. PLoS ONE 2013 8(7): e69581
R. Singh, E. van Nood, M. Nieuwdorp, B. van Dam, I.J. Ten Berge, S.E. Geerlings, F.J. Bemelman. Donor feces infusion for eradication of Extended Spectrum beta-Lactamase producing Escherichia coli in a patient with end stage renal disease. Clin Microbiol Infect. Volume 20, Issue 11, pages 0977-0978
T Urich, A Lanzén, J Qi, et al.. Simultaneous assessment of soil microbial community structure and function through analysis of the meta-transcriptome. PLoS One 2008 3:e2527
C. Ubeda, V Bucci, S. Caballero, Ana Djukovic, Nora C. Toussaint, Michele Equinda, Lauren Lipuma, Lilan Ling, Asia Gobourne, Daniel No, Ying Taur, Robert R. Jenq, Marcel R. M. van den Brink, Joao B. Xavier and Eric G. Pamer. Intestinal Microbiota Containing Barnesiella Species Cures Vancomycin-Resistant Enterococcus faecium Colonization. Infect. Immun. March 2013 vol. 81 no. 3 965-973.

## Claims

1. An *in vitro* method for assessing the risk to develop intestinal carriage of extended-spectrum beta-lactamase producing Enterobacteriaceae (E-ESBL) or for prognosing intestinal carriage of E-ESBL, comprising a step of determining the abundance, in the gut microbiota of a subject, of at least one bacterium selected from the list consisting of bacteria from the genus *Desulfovibrio, Oscillospira, Parabacteroides, Coprococcus* and *Prevotella.*

2. The method according to claim 1, wherein it comprises a step of determining the abundance of all the bacteria of at least one genus selected from the list consisting of the genus *Desulfovibrio, Oscillospira, Parabacteroides, Coprococcus* and *Prevotella.*

3. The method according to claim 1 or 2, wherein the abundance of said at least one bacterium is determined from a sample of feces from said subject.

4. The method according to any of claims 1 to 3, wherein the abundance of said at least one bacterium is determined in the total gut microbiota of said subject.

5. The method according to any of claims 1 to 3, wherein the abundance of said at least one bacterium is determined in the active gut microbiota of said subject.

6. The method according to any of claims 1 to 5, wherein said subject is an animal, preferably a mammal or an aves.

7. The method according to any of claims 1 to 6, wherein said subject is a human being.

8. The method according to any of claims 1 to 7, wherein it comprises a step of measuring the concentration of 16S rDNA or 16S rRNA of said bacterium.

9. A mucosal composition comprising at least one bacterium selected from the list consisting of bacteria from the genus *Desulfovibrio, Oscillospira, Parabacteroides,* and *Coprococcus.*

10. The mucosal composition according to claim 9, wherein it further comprises a pharmaceutical carrier.

11. The mucosal composition according to claim 9 or 10, wherein it further comprises a prebiotic.

12. At least one bacterium selected from the list consisting of bacteria from the genus *Desulfovibrio, Oscillospira, Parabacteroides,* and *Coprococcus,* or a composition comprising same, for use in the prevention or treatment of intestinal carriage of extended-spectrum beta-lactamase producing Enterobacteriaceae in a subject in need thereof.

13. The bacterium or composition comprising same for the use according to claim 12, wherein said subject is a human being.
